# EUROPEAN PATENT APPLICATION

(11) **EP 4 599 692 A1**
(43) Date of publication of application: **13.08.2025**
(21) Application number: 23874807.3
(22) Date of filing: 02.10.2023
(51) Int. Cl.: A23L 5/00, A23F 5/28, A23L 33/105

(54) **ORAL COMPOSITION**

(30) Priority: 03.10.2022 JP 2022159652
(71) Applicant: Kao Corporation, Chuo-ku, Tokyo 103-8210 (JP)
(72) Inventor: KOBAYASHI, Yusuke, Tokyo 131-8501 (JP); SHIMODA, Yuichi, Tokyo 131-8501 (JP)
(74) Representative: Hoffmann Eitle
(86) International application number: PCT/JP2023/035875
(87) International publication number: WO 2024/075680

(57) **Abstract**

Provided is an oral composition, including the following components (A), (B), and (C): (A) 5 mass% to 20 mass% of a chlorogenic acid; (B) 0.001 mass% or more and less than 0.4 mass% of caffeine; and (C) 4-vinylguaiacol, wherein a mass ratio of the component (C) to the component (B), [(C)/(B)], is 3×10⁻⁴ or more and 1100×10⁻⁴ or less.

## Description

### Field of the Invention

The present invention relates to an oral composition.

### Background of the Invention

A chlorogenic acid is a kind of polyphenol, and has been reported to have physiological actions, such as an antioxidant action and a hypotensive action. A coffee bean has been known as a material containing a large amount of the chlorogenic acid. The higher the roasting degree of the coffee bean becomes, the richer its aroma becomes, and hence its preference is improved. However, when the roasting degree of the coffee bean is increased, a considerable amount of the chlorogenic acid present in the coffee bean is decomposed. Accordingly, to take full advantage of the chlorogenic acid, it is advantageous to use a chlorogenic acid reagent, or a coffee bean with a low roasting degree or an unroasted coffee bean as a raw material. However, when the reagent or these coffee beans are used as a raw material, harshness derived from the chlorogenic acid is so strong as to be liable to constitute an obstacle in continuous ingestion. For example, the incorporation of 4-vinylguaiacol at a specific mass ratio with respect to the chlorogenic acid has been known as a technology of suppressing such harshness derived from the chlorogenic acid (Patent Document 1).

In addition, the following has been reported as a technology of improving the taste of a preference beverage: in, for example, a beverage having a low acidity and containing caffeine, the incorporation of a predetermined amount of β-damascenone and guaiacol can suppress bitterness caused by caffeine (Patent Document 2). Further, it has been reported that the body (i.e., mouthfeel) of a packaged coffee beverage can be enhanced as follows (Patent Document 3): a roasted coffee bean is vaporized by adding water thereto and heating the mixture; a vaporized fraction containing furfuryl alcohol and γ-butyrolactone is concentrated and collected; and the collected fraction is added to the beverage.

[Patent Document 1] JP-A-2022-65462
[Patent Document 2] JP-A-2019-213512
[Patent Document 3] WO 2010/147222 A1

### Summary of the Invention

The present invention provides the following [1] to [12].
[1] An oral composition, comprising the following components (A), (B), and (C):
   (A) 5 mass% to 20 mass% of a chlorogenic acid;
   (B) 0.001 mass% or more and less than 0.4 mass% of caffeine; and
   (C) 4-vinylguaiacol,
   wherein a mass ratio of the component (C) to the component (B), [(C)/(B)], is 3×10⁻⁴ or more and 1100×10⁻⁴ or less.
[2] The oral composition according to [1], wherein a mass ratio of the component (C) to the component (A), [(C)/(A)], is 1×10⁻⁶ or more and 300×10⁻⁶ or less.
[3] The oral composition according to [1] or [2], wherein a mass ratio of the component (B) to the component (A), [(B)/(A)], is 1×10⁻⁴ or more and 500×10⁻⁴ or less.
[4] The oral composition according to any one of [1] to [3], wherein a content of the component (C) is from 0.1 mass ppm to 60 mass ppm.
[5] The oral composition according to any one of [1] to [4], further comprising a lactone as a component (D).
[6] The oral composition according to [5], wherein the component (D) comprises γ-butyrolactone.
[7] The oral composition according to [5] or [6], wherein a mass ratio of the component (D) to the component (B), [(D)/(B)], is 3×10⁻⁴ or more and 1200×10⁻⁴ or less.
[8] The oral composition according to any one of [5] to [7], wherein a mass ratio of the component (D) to the component (C), [(D)/(C)], is from 0.1 to 50.
[9] The oral composition according to any one of [1] to [8], wherein the oral composition is a solid oral composition.
[10] The oral composition according to any one of [1] to [8], wherein the oral composition is a liquid oral composition.
[11] An astringency suppressant for an oral product, comprising the following components (A) and (B), the astringency suppressant comprising (C) 4-vinylguaiacol as an active ingredient:
   (A) 5 mass% to 20 mass% of a chlorogenic acid; and
   (B) 0.001 mass% or more and less than 0.4 mass% of caffeine,
   wherein (C) 4-vinylguaiacol is caused to coexist at such a ratio that a mass ratio of (C) 4-vinylguaiacol to (B) caffeine, [(C)/(B)], becomes 3×10⁻⁴ or more and 1100×10⁻⁴ or less.
[12] A method of suppressing astringency of an oral product including the following components (A) and (B):
   (A) 5 mass% to 20 mass% of a chlorogenic acid; and
   (B) 0.001 mass% or more and less than 0.4 mass% of caffeine,
   the method comprising causing (C) 4-vinylguaiacol to coexist at such a ratio that a mass ratio of (C) 4-vinylguaiacol to (B) caffeine, [(C)/(B)], becomes 3×10⁻⁴ or more and 1100×10⁻⁴ or less.

### Detailed Description of the Invention

The present inventors made an investigation with a view to developing an oral composition in which the physiological effect of a chlorogenic acid was enhanced through use of a chlorogenic acid reagent, or a coffee bean with a low roasting degree or an unroasted coffee bean as a raw material. As a result, the inventors found that when caffeine was incorporated into a high concentration of the chlorogenic acid, different types of taste behavior appeared depending on caffeine contents. That is, the present inventors found that when a trace amount of caffeine was incorporated into a high concentration of the chlorogenic acid, astringency was sensed.

The present invention relates to an oral composition suppressed in astringency.

In view of the above-mentioned problem, the present inventors made extensive investigations. As a result, the inventors found that in an oral composition containing a high concentration of a chlorogenic acid and a trace amount of caffeine, the incorporation of 4-vinylguaiacol at a specific mass ratio with respect to caffeine was able to suppress its astringency. Further, the present inventors found that the incorporation of a lactone in combination with 4-vinylguaiacol was able to enhance the astringency-suppressing effect.

According to the present invention, the oral composition suppressed in astringency can be provided.

### [Oral Composition]

The oral composition of the present invention contains a chlorogenic acid as a component (A). As used herein, the term "chlorogenic acid" is a generic term including: monocaffeoylquinic acids, such as 3-caffeoylquinic acid, 4-caffeoylquinic acid, and 5-caffeoylquinic acid; monoferuloylquinic acids, such as 3-feruloylquinic acid, 4-feruloylquinic acid, and 5-feruloylquinic acid; and dicaffeoylquinic acids, such as, 3,4-dicaffeoylquinic acid, 3,5-dicaffeoylquinic acid, and 4,5-dicaffeoylquinic acid. In the present invention, at least one kind out of the above-mentioned nine kinds has only to be incorporated. The component (A) may be in the form of a salt or a hydrate. While the salt is not particularly limited as long as the salt is physiologically acceptable, the salt may be, for example, an alkali metal salt.

The product from which the component (A) is derived is not particularly limited as long as the product is typically used in the field of a food and beverage. For example, the component may be a chemically synthesized product or a naturally derived product. The naturally derived product may be, for example, a plant extract. While a plant to be used in extraction is not particularly limited as long as the plant contains the component (A), examples thereof include a sunflower seed, an apple unripe fruit, a coffee bean, a simon leaf, a cone of a Pinaceae plant, a seed husk of a Pinaceae plant, sugarcane, a heavenly bamboo leaf, a burdock, the skin of an eggplant, a Japanese apricot fruit, a coltsfoot, and a Vitaceae plant. The plants may be used alone or in combination thereof. A method for the extraction of the plant extract and conditions for the extraction are not particularly limited, and a known method may be adopted. In addition, the plant extract may be concentrated or dried, and may be purified for improving the purity of the chlorogenic acid. A known method has only to be adopted as a method for each of the concentration, the drying, and the purification.

While the content of the component (A) in the oral composition of the present invention is from 5 mass% to 20 mass%, the content is preferably 6 mass% or more, more preferably 6.5 mass% or more, more preferably 7 mass% or more from the viewpoint of enhancing the physiological effect of the component (A). In addition, from the viewpoint of suppressing the astringency of the composition, the content is preferably 18 mass% or less, more preferably 17 mass% or less, more preferably 16 mass% or less. Moreover, the content of the component (A) in the oral composition of the present invention is preferably from 6 mass% to 18 mass%, more preferably from 6.5 mass% to 17 mass%, more preferably from 7 mass% to 16 mass%. As used herein, the content of the component (A) is defined on the basis of the total amount of the above-mentioned nine kinds of the chlorogenic acid. When the component (A) is in the form of a salt or a hydrate, the content of the component (A) is a value in terms of a chlorogenic acid that is a free acid. The content of the component (A) may be measured by an analysis method suitable for the state of a measurement sample among generally known measurement methods, and for example, may be analyzed by liquid chromatography. A specific example thereof is a method described in Examples to be described later. At the time of the measurement of the content, for example, such treatment as described below may be appropriately performed as required: the sample is lyophilized so that the sample may be adapted to the detection range of an apparatus; or contaminants in the sample are removed so that the sample may be adapted to the resolution of the apparatus.

The oral composition of the present invention contains caffeine as a component (B). The component (B) may be derived from a raw material or newly added.

While the content of the component (B) in the oral composition of the present invention is 0.001 mass% or more and less than 0.4 mass%, the content is preferably 0.005 mass% or more, more preferably 0.03 mass% or more, more preferably 0.05 mass% or more from the viewpoint of a range in which the effect of the present invention is easily obtained. In addition, from the same viewpoint, the content is preferably 0.3 mass% or less, more preferably 0.25 mass% or less, more preferably 0.2 mass% or less, even more preferably 0.15 mass% or less. In addition, the content of the component (B) in the oral composition of the present invention is preferably from 0.005 mass% to 0.3 mass%, more preferably from 0.005 mass% to 0.25 mass%, more preferably from 0.03 mass% to 0.2 mass%, even more preferably from 0.05 mass% to 0.15 mass%. The content of the component (B) may be measured by an analysis method suitable for the state of a measurement sample among generally known measurement methods, and for example, may be analyzed by liquid chromatography. A specific example thereof is a method described in Examples to be described later. At the time of the measurement of the content, for example, such treatment as described below may be appropriately performed as required: the sample is lyophilized so that the sample may be adapted to the detection range of an apparatus; or contaminants in the sample are removed so that the sample may be adapted to the resolution of the apparatus.

In the oral composition of the present invention, from the viewpoint of suppressing its astringency, the mass ratio of the component (B) to the component (A), [(B)/(A)], is preferably 1×10⁻⁴ or more, more preferably 3×10⁻⁴ or more, more preferably 5×10⁻⁴ or more, and is preferably 500×10⁻⁴ or less, more preferably 300×10⁻⁴ or less, more preferably 100×10⁻⁴ or less. In addition, the above-mentioned mass ratio [(B)/(A)] in the oral composition of the present invention is preferably 1×10⁻⁴ or more and 500×10⁻⁴ or less, more preferably 3×10⁻⁴ or more and 300×10⁻⁴ or less, more preferably 5×10⁻⁴ or more and 100×10⁻⁴ or less.

The oral composition of the present invention contains 4-vinylguaiacol as a component (C). Herein, "4-vinylguaiacol" has been known not only as a substance inhibiting the sharpness of an aftertaste in a roasted coffee beverage but also as a substance causing a foreign odor, such as a smoke odor, a medicinal odor, or a spice odor, in sake. In the present invention, however, it was found that when the component (C) was incorporated so that the mass ratio of the component (C) to the component (B) fell within a specific range, surprisingly, astringency specifically occurring at the time of the incorporation of a trace amount of caffeine into a high concentration of a chlorogenic acid was able to be suppressed. The component (C) may be derived from a raw material or newly added.

While the mass ratio of the component (C) to the component (B) in the oral composition of the present invention, [(C)/(B)], is 3×10⁻⁴ or more and 1 100×10⁻⁴ or less, from the viewpoint of suppressing its astringency, the ratio is preferably 4×10⁻⁴ or more, more preferably 6×10⁻⁴ or more, more preferably 8×10⁻⁴ or more. In addition, from the viewpoint of suppressing a medicinal odor derived from 4-vinylguaiacol, the ratio is preferably 400×10⁻⁴ or less, more preferably 250×10⁻⁴ or less, more preferably 150×10⁻⁴ or less. Moreover, the above-mentioned mass ratio [(C)/(B)] in the oral composition of the present invention is preferably 4×10⁻⁴ or more and 400×10⁻⁴ or less, more preferably 6×10⁻⁴ or more and 250×10⁻⁴ or less, more preferably 8×10⁻⁴ or more and 150×10⁻⁴ or less. The mass ratio [(C)/(B)] is calculated after the respective contents of the component (B) and the component (C) are converted into the same unit.

In addition, in the oral composition of the present invention, from the viewpoint of suppressing its astringency, the mass ratio of the component (C) to the component (A), [(C)/(A)], is preferably 1×10⁻⁶ or more, more preferably 3×10⁻⁶ or more, more preferably 5×10⁻⁶ or more, even more preferably 40×10⁻⁶ or more. In addition, from the viewpoint of suppressing the medicinal odor derived from 4-vinylguaiacol, the ratio is preferably 300×10⁻⁶ or less, more preferably 180×10⁻⁶ or less, more preferably 120×10⁻⁶ or less, even more preferably 80×10⁻⁶ or less. Moreover, the above-mentioned mass ratio [(C)/(A)] in the oral composition of the present invention is preferably 1×10⁻⁶ or more and 300×10⁻⁶ or less, more preferably 3×10⁻⁶ or more and 180×10⁻⁶ or less, more preferably 5×10⁻⁶ or more and 120×10⁻⁶ or less, even more preferably 40×10⁻⁶ or more and 80×10⁻⁶ or less. The mass ratio [(C)/(A)] is calculated after the respective contents of the component (A) and the component (C) are converted into the same unit.

The content of the component (C) in the oral composition of the present invention is preferably 0.1 mass ppm or more, more preferably 0.3 mass ppm or more, more preferably 0.7 mass ppm or more, even more preferably 6 mass ppm or more from the viewpoint of suppressing its astringency. In addition, from the viewpoint of suppressing the medicinal odor derived from 4-vinylguaiacol, the content is preferably 60 mass ppm or less, more preferably 25 mass ppm or less, more preferably 20 mass ppm or less, even more preferably 15 mass ppm or less. Moreover, the content of the component (C) in the oral composition of the present invention is preferably from 0.1 mass ppm to 60 mass ppm, more preferably from 0.3 mass ppm to 25 mass ppm, more preferably from 0.7 mass ppm to 20 mass ppm, even more preferably from 6 mass ppm to 15 mass ppm. The content of the component (C) may be measured by an analysis method suitable for the state of a measurement sample among generally known measurement methods, and may be measured by, for example, a GC/MS method. A specific example thereof may be a method described in Examples to be described later. At the time of the measurement, for example, such treatment as described below may be appropriately performed as required: the sample is lyophilized so that the sample may be adapted to the detection range of an apparatus; or contaminants in the sample are removed so that the sample may be adapted to the resolution of the apparatus.

Further, the oral composition of the present invention may contain a lactone as a component (D). The lactone has been known as a compound having a unique odor. The present inventors found that the incorporation of the lactone alone was not able to suppress astringency specifically occurring at the time of the incorporation of a trace amount of caffeine into a high concentration of a chlorogenic acid, but an astringency-suppressing effect was able to be enhanced by the incorporation of the lactone in combination with 4-vinylguaiacol. The component (D) may be derived from a raw material or newly added.

While the component (D) is not particularly limited as long as the component is a lactone, from the viewpoint of suppressing the astringency of the oral composition, the component is preferably one or more selected from the group consisting of γ-butyrolactone, γ-decalactone, and δ-decanolactone, more preferably γ-butyrolactone.

The mass ratio of the component (D) to the component (B) in the oral composition of the present invention, [(D)/(B)], is preferably 3×10⁻⁴ or more, more preferably 8×10⁻⁴ or more, more preferably 20×10⁻⁴ or more from the viewpoint of suppressing its astringency. In addition, from the viewpoint of suppressing a foreign odor derived from the lactone, the ratio is preferably 1 200×10⁻⁴ or less, more preferably 500×10⁻⁴ or less, more preferably 300×10⁻⁴ or less. Moreover, the above-mentioned mass ratio [(D)/(B)] in the oral composition of the present invention is preferably 3×10⁻⁴ or more and 1 200×10⁻⁴ or less, more preferably 8×10⁻⁴ or more and 500×10⁻⁴ or less, more preferably 20×10⁻⁴ or more and 300×10⁻⁴ or less. The mass ratio [(D)/(B)] is calculated after the respective contents of the component (B) and the component (D) are converted into the same unit.

The mass ratio of the component (D) to the component (C) in the oral composition of the present invention, [(D)/(C)], is preferably 0.1 or more, more preferably 0.5 or more, more preferably 1.5 or more from the viewpoint of suppressing its astringency. In addition, from the viewpoint of suppressing a foreign odor derived from the lactone, the ratio is preferably 50 or less, more preferably 25 or less, more preferably 15 or less. Moreover, the above-mentioned mass ratio [(D)/(C)] in the oral composition of the present invention is preferably from 0.1 to 50, more preferably from 0.5 to 25, more preferably from 1.5 to 15.

The content of the component (D) in the oral composition of the present invention is preferably 0.1 mass ppm or more, more preferably 0.5 mass ppm or more, more preferably 1.5 mass ppm or more from the viewpoint of suppressing its astringency. In addition, from the viewpoint of suppressing a foreign odor derived from the lactone, the content is preferably 90 mass ppm or less, more preferably 60 mass ppm or less, more preferably 40 mass ppm or less. Moreover, the content of the component (D) in the oral composition of the present invention is preferably from 0.1 mass ppm to 90 mass ppm, more preferably from 0.5 mass ppm to 60 mass ppm, more preferably from 1.5 mass ppm to 40 mass ppm. The content of the component (D) may be measured by an analysis method suitable for the state of a measurement sample among generally known measurement methods, and may be measured by, for example, a GC/MS method. A specific example thereof may be a method described in Examples to be described later. At the time of the measurement, such treatment as described below may be appropriately performed as required: the sample is lyophilized so that the sample may be adapted to the detection range of an apparatus; or contaminants in the sample are removed so that the sample may be adapted to the resolution of the apparatus.

The oral composition of the present invention may contain one or more of additives, such as a sweetener, an acidulant, an amino acid, a protein, a vitamin, a mineral, a perfume, fruit juice, a plant extract, an ester, a coloring matter, an emulsifier, a milk component, cocoa powder, a seasoning, a vegetable oil and fat, an antioxidant, a preservative, a pH adjuster, a gelling agent, and a carrier, as desired. The content of the additive may be appropriately set within a range that does not impair the purpose of the present invention.

The term "oral composition" as used herein refers to a composition that is unlikely to cause harm to human health and is exclusively orally ingested in a general social life, and the composition is not limited by divisions, such as a food, a pharmaceutical, and a quasi-drug, in administrative divisions. Accordingly, the oral composition of the present invention encompasses a composition encompassing a wide variety of foods and beverages for forming, for example, a general food, a health food (functional food and beverage), a food with health claims (a food for specified health use, a food with nutrient function claims, or a food with function claims), a quasi-drug, and a pharmaceutical to be orally ingested.

The oral composition of the present invention may be in a liquid form or a solid form at normal temperature (20°C±15°C), and may adopt any appropriate form.

The oral composition of the present invention is preferably used for an oral composition excluding a coffee beverage. The coffee beverage may be clearly distinguished as a product from the oral composition of the present invention because the content of furfuryl mercaptan (hereinafter referred to as "component (E)") in the coffee beverage is typically 0.00006 mass% or more. That is, the content of the component (E) in the oral composition of the present invention is less than 0.00006 mass%, preferably less than 0.00003 mass%, more preferably less than 0.00001 mass%, and the composition is more preferably substantially free of the component (E). As used herein, the phrase "substantially free of" is a concept encompassing not only a case in which the component (E) is completely absent in the oral composition of the present invention but also a case in which its concentration is less than a detection limit. The content of the component (E) may be measured by an analysis method suitable for the state of a measurement sample among generally known measurement methods, and may be measured by, for example, a GC/MS method. A specific example thereof may be a method described in Examples to be described later. At the time of the measurement, such treatment as described below may be appropriately performed as required: the sample is lyophilized so that the sample may be adapted to the detection range of an apparatus; or contaminants in the sample are removed so that the sample may be adapted to the resolution of the apparatus.

When a naturally derived product is used as the component (A) in the oral composition of the present invention, a lightly roasted coffee bean or a green coffee bean is preferred from the viewpoint of the content of the chlorogenic acid. As used herein, the term "lightly roasted coffee bean" refers to a roasted coffee bean having an L value of 30 or more and 60 or less. From the viewpoint of the physiological effect of the component (A), the L value of the lightly roasted coffee bean is preferably 32 or more, more preferably 34 or more, more preferably 36 or more, more preferably 38 or more, even more preferably 40 or more. The term "L value" as used herein refers to a value obtained by measuring the brightness of a roasted coffee bean with a color-difference meter while setting a black color and a white color to an L value of 0 and an L value of 100, respectively. The kind and production site of the coffee bean are not particularly limited. In addition, coffee beans different from each other in kind or production site may be used alone or in combination thereof.

Meanwhile, the coffee beverage typically uses, as a raw material, roasted coffee beans including a roasted coffee bean having an L value of less than 30. Accordingly, the oral composition of the present invention is a concept that does not encompass an oral composition (e.g., a coffee beverage) using, as a raw material, roasted coffee beans including a roasted coffee bean having an L value of preferably less than 30, more preferably less than 32, more preferably less than 34, even more preferably less than 36, even more preferably less than 38, even more preferably less than 40. The content of a coffee content in the coffee beverage includes a coffee content extracted or eluted from 1 g or more of roasted coffee beans in terms of green coffee beans in 100 g of a net content. The "value in terms of green beans" as used herein encompasses that 1 g of roasted coffee beans correspond to 1.3 g of green coffee beans (Newly Revised Edition·Soft Drinks, supervision: Japan Soft Drink Association, publication: Korin, published on December 25, 1989, described on page 421). In addition, the kind of the coffee beverage is not particularly limited, but examples thereof may include coffee beverages and the like defined in Article 2 of "Fair Competition Code on Labeling of Coffee Beverages and the Like" amended and enforced on August 19, 2019, that is, "coffee", a "coffee beverage," a "coffee-containing soft drink," and a "coffee-containing carbonated beverage."

The components (B) to (D) may each be a chemically synthesized product (e.g., a reagent) or a naturally derived product. The naturally derived product may, for example, a plant, and an extract is permitted. A plant containing the component (B) may, for example, a coffee bean or a tea leaf, but is not limited thereto. In addition, a plant containing the component (C) may, for example, a coffee bean, rice bran, or the bran of a wheat variety, but is not limited thereto. Further, a plant containing the component (D) may, for example, a coffee bean, a grape, or the pulp of an apricot, but is not limited thereto. The plants may be used alone or in combination thereof. A method for the extraction of the plant extract and conditions for the extraction are not particularly limited, and a known method may be adopted. In addition, the plant extract may be a concentrated product or a dried product.

A suitable aspect of the oral composition of the present invention may be, for example, a solid oral composition or a liquid oral composition. The respective compositions are described below in conformity with preferred embodiments.

The form of the solid oral composition of the present invention is not particularly limited as long as the composition is a solid at normal temperature (20°C±15°C), and examples thereof may include various forms, such as a powder form, a granule form, a tablet form, a rod form, a plate form, and a block form. A solid content in the solid oral composition of the present invention is typically 90 mass% or more, preferably 93 mass% or more, more preferably 95 mass% or more, more preferably 97 mass% or more. The upper limit of such solid content is not particularly limited, and the solid content may be 100 mass%. As used herein, the term "solid content" refers to the mass of a residue obtained by drying 1 g of a sample in an electric thermostat dryer at 105°C for 3 hours to remove its volatile substance.

Examples of the solid oral composition of the present invention may include a food, a pharmaceutical, and a quasi-drug. Of those, a solid food is preferred because the effect of the present invention is easily obtained, and a powder food is more preferred.

When the solid oral composition of the present invention is a solid food, examples thereof may include: confectionery, such as drop, candy, gum, chocolate, and cookie bread; and health/beauty/nutritional auxiliary food such as a supplement.

In addition, when the solid oral composition of the present invention is a pharmaceutical or a quasi-drug, examples of the dosage form thereof may include a granule, a powder, a tablet, a pill, a chewable, and a troche. In addition, when the composition is used as a tablet, a dividable tablet having formed therein a dividing line is permitted.

Of those, a supplement, a powder, a tablet, or a granule is preferred as the solid oral composition.

The solid oral composition of the present invention may contain an acceptable carrier as required so as to be formed into a solid form. Examples thereof may include carriers including: excipients (e.g., starches, such as waxy corn starch, sweet potato starch, and potato starch; sugar alcohols, such as xylitol, sorbitol, maltitol, lactitol, reduced palatinose, trehalose, and palatinose; lactose; oligosaccharides; crystalline cellulose; light silicic anhydride; and calcium hydrogen phosphate); binders (e.g., gelatin, pregelatinized starch, polyvinylpyrrolidone, polyvinyl alcohol, pullulan, and hydrogenated oils); disintegrants (e.g., carmellose, carmellose calcium, croscarmellose sodium, and crospovidone); lubricants (e.g., calcium stearate, magnesium stearate, sucrose fatty acid esters, sodium stearyl fumarate, talc, and silicon dioxide); taste-making agents (e.g., stevia); extenders; surfactants; dispersants; buffers; antioxidants; preservatives; quality stabilizers; and diluents.

In addition, the solid oral composition of the present invention may be an instant beverage composition. As used herein, the term "instant beverage composition" refers to a composition that is diluted with a liquid in accordance with a predetermined usage method and orally ingested as a reconstituted beverage. The liquid is not particularly limited as long as the liquid can reconstitute the composition into a beverage. Examples of the liquid may include water, carbonated water, cow's milk, and soy milk. The temperature of the liquid is not limited. A dilution factor, which has only to follow a predetermined usage method, is typically from 30 mass-fold to 800 mass-fold, preferably from 80 mass-fold to 600 mass-fold.

The solid oral composition of the present invention may be produced in accordance with a conventional method, and an appropriate method may be adopted. The composition may be produced by, for example, mixing the components (A) to (C), and as required, any other component so that the respective contents of the component (A) and the component (B), and the mass ratio of the component (C) to the component (B), [(C)/(B)], may fall within the above-mentioned ranges. The order in which the components (A) to (C), and the other component are mixed is not particularly limited, and the components may be mixed in any order. While an appropriate method, such as stirring or shaking, may be adopted as a method for the mixing, a mixing apparatus may be used. The mixing system of the mixing apparatus may be of a rotating vessel type or a fixed vessel type. As the rotating vessel type, for example, a horizontal cylinder type, a V type, a double-cone type, or a cubic type may be adopted. In addition, as the fixed vessel type, for example, a ribbon type, a screw type, a conical screw type, a paddle type, a fluidized bed type, or a Phillips blender may be adopted.

In addition, the solid oral composition of the present invention may be produced as a granulated product by a known granulation method. Examples of the granulation method may include spray granulation, fluidized bed granulation, compression granulation, tumbling granulation, stirring granulation, extrusion granulation, and powder coating granulation. Granulation conditions may be appropriately selected in accordance with the granulation method. In addition, when the solid oral composition is produced as a tablet, any of wet tableting and dry tableting may be adopted, and a known compression molding machine may be used.

The solid oral composition of the present invention may be loaded into a package. Examples of the package may include a bottle, a can, a bottle, a box-type container, a stick-type package, and a pillow-type package. When the solid oral composition of the present invention is loaded into the package, a commercial loading machine may be used. For example, small portions each corresponding to one intake of the solid oral composition of the present invention may be individually packaged. When the solid oral composition is an instant beverage composition, the composition may be, for example, any one of the following: a product that is filled in a container such as a bottle and is measured with a spoon or the like in an amount corresponding to a cup before drinking; a cup type accommodating an amount corresponding to a cup; and a stick type in which small portions each corresponding to a cup are individually packaged. The inside of the container and the inside of a packaging material may be filled with a nitrogen gas, and the packaging material preferably has low oxygen permeability from the viewpoint of quality maintenance.

The form of the liquid oral composition of the present invention is not particularly limited as long as the composition has fluidity at normal temperature (20°C±15°C). Examples thereof may include a liquid, a concentrated liquid form, a gel form, and a jelly form.

Examples of the product form of the liquid oral composition of the present invention may include: a ready-to-drink (RTD)-type beverage composition; dairy products, such as yogurt, processed milk, and fermented milk; an oil and fat, and an oil and fat processed food, such as a salad oil, a tempura oil, margarine, mayonnaise, shortening, whipped cream, and dressing; seasonings, such as sauce and tare sauce; and health/beauty/nutritional auxiliary food such as a drinkable preparation. As used herein, the term "RTD-type beverage composition" refers to a beverage that can be drunk as it is without being diluted.

Of those, an RTD-type beverage composition is preferred as the liquid oral composition. Examples of the form of the RTD-type beverage composition may include a liquid, a concentrated liquid form, a gel form, and a jelly form. When the form is a concentrated liquid form, a gel form, or a jelly form, the beverage composition has only to be capable of being sucked from a suction opening or a straw attached to its container, and its solid content concentration is not particularly limited.

From the viewpoint of its taste and flavor, the pH (20°C) of the RTD-type beverage composition is preferably 3 or more, more preferably 3.5 or more, more preferably 4 or more, and is preferably 7 or less, more preferably 6.5 or less, more preferably 6 or less. The pH is measured with a pH meter after the temperature of the composition has been adjusted to 20°C.

The RTD-type beverage composition may be a non-alcoholic beverage or an alcoholic beverage. As used herein, the term "non-alcoholic beverage" refers to a beverage having an alcohol concentration of less than 1 v/v%, and encompasses a beverage that is completely free of any alcohol and a beverage having an alcohol concentration of 0.00 v/v%. The term "alcohol" as used herein means ethanol unless otherwise stated.

Examples of the non-alcoholic beverage may include a tea beverage, a carbonated beverage, a fruit juice beverage, a vegetable beverage, a dairy beverage, a sports beverage, an isotonic beverage, enhanced water, bottled water, near water, a dietary drinkable preparation, and a beauty drinkable preparation.

Examples of the alcoholic beverage may include beer, wine, sake, plum liquor, sparkling liquor, whiskey, brandy, Japanese distilled spirit, rum, gin, and liqueurs.

The RTD-type beverage composition may be packed in a container. The container is not particularly limited as long as the container is a typical packaging container, and examples thereof may include: a molded container containing polyethylene terephthalate as a main component (so-called plastic bottle); a metal can; a paper container composited with metal foil or a plastic film; and a bottle.

When the RTD-type beverage composition is a packaged beverage composition, the composition may have already been sterilized by heating. A method for the heat sterilization is not particularly limited as long as the method conforms to conditions specified in laws and regulations to be applied (the Food Sanitation Act in Japan).

The liquid oral composition of the present invention may be produced in accordance with a conventional method, and any appropriate method may be adopted. The composition may be produced by, for example, mixing the components (A) to (C), and as required, any other component together with a liquid so that the respective contents of the component (A) and the component (B), and the mass ratio of the component (C) to the component (B), [(C)/(B)], may fall within the above-mentioned ranges. The order in which the components (A) to (C), and the other component are mixed is not particularly limited, and the components may be mixed in any order. Examples of the liquid may include water, carbonated water, cow's milk, and soy milk. The temperature of the liquid is not limited.

### [Astringency Suppressant and Method of suppressing Astringency]

An astringency suppressant of the present invention is an astringency suppressant for an oral product, containing 5 mass% to 20 mass% of (A) a chlorogenic acid, and 0.001 mass% or more and less than 0.4 mass% of (B) caffeine, the astringency suppressant containing (C) 4-vinylguaiacol as an active ingredient, wherein (C) 4-vinylguaiacol is caused to coexist at such a ratio that the mass ratio of (C) 4-vinylguaiacol to (B) caffeine, [(C)/(B)], becomes 3×10⁻⁴ or more and 1 100×10⁻⁴ or less.

In addition, a method of suppressing astringency of the present invention is a method of suppressing astringency of an oral product including 5 mass% to 20 mass% of (A) a chlorogenic acid, and 0.001 mass% or more and less than 0.4 mass% of (B) caffeine, the method containing causing (C) 4-vinylguaiacol to coexist at such a ratio that the mass ratio of (C) 4-vinylguaiacol to (B) caffeine, [(C)/(B)], becomes 3×10⁻⁴ or more and 1 100×10⁻⁴ or less.

The oral product is not particularly limited as long as the product can be orally ingested, and the product may be in a liquid form or a solid form. The oral product may be, for example, a food and beverage, a pharmaceutical, or a quasi-drug containing the component (A) and the component (B). Of those, a food and beverage is preferred.

Examples of the food and beverage may include: a beverage or instant beverage containing the component (A) and the component (B); and a food containing the components (A) and (B). The food and beverage may be produced in accordance with a conventional method depending on the kind of the food and beverage.

The respective contents of the component (A) and the component (B) in the oral product, and the respective mass ratios between the components are as described above.

In addition, to enhance a suppressing effect on the astringency of the oral product, (D) a lactone may be caused to coexist together with (C) 4-vinylguaiacol. A specific aspect of the component (D), the content of the component (D) in the oral product, and mass ratios between the components are as described above.

The dosage form of each of the pharmaceutical and the quasi-drug is not particularly limited, and may, for example, a preparation for oral administration. For example, a known dosage form, such as a solution or syrup, may be adopted. In addition, when the pharmaceutical or the quasi-drug is turned into the preparation, a known additive may be blended. The pharmaceutical and the quasi-drug may each be produced in accordance with a conventional method.

In relation to the above-mentioned embodiments, the present invention further discloses the following aspects.

<1> An oral composition, comprising the following components (A), (B), and (C):
   (A) 5 mass% to 20 mass% of a chlorogenic acid;
   (B) 0.001 mass% or more and less than 0.4 mass% of caffeine; and
   (C) 4-vinylguaiacol,
   wherein a mass ratio of the component (C) to the component (B), [(C)/(B)], is 3×10⁻⁴ or more and 1 100×10⁻⁴ or less.
<2> The oral composition according to <1>, wherein a content of the component (A) is preferably 6 mass% or more, more preferably 6.5 mass% or more, more preferably 7 mass% or more, and is preferably 18 mass% or less, more preferably 17 mass% or less, more preferably 16 mass% or less.
<3> The oral composition according to <1>, wherein a content of the component (A) is preferably from 6 mass% to 18 mass%, more preferably from 6.5 mass% to 17 mass%, more preferably from 7 mass% to 16 mass%.
<4> The oral composition according to any one of <1> to <3>, wherein a content of the component (B) is preferably 0.005 mass% or more, more preferably 0.03 mass% or more, more preferably 0.05 mass% or more, and is preferably 0.3 mass% or less, more preferably 0.25 mass% or less, more preferably 0.2 mass% or less, even more preferably 0.15 mass% or less.
<5> The oral composition according to any one of <1> to <3>, wherein a content of the component (B) is preferably from 0.005 mass% to 0.3 mass%, more preferably from 0.005 mass% to 0.25 mass%, more preferably from 0.03 mass% to 0.2 mass%, even more preferably from 0.05 mass% to 0.15 mass%.
<6> The oral composition according to any one of <1> to <5>, wherein a mass ratio of the component (B) to the component (A), [(B)/(A)], is preferably 1×10⁻⁴ or more, more preferably 3×10⁻⁴ or more, more preferably 5×10⁻⁴ or more, and is preferably 500×10⁻⁴ or less, more preferably 300×10⁻⁴ or less, more preferably 100×10⁻⁴ or less.
<7> The oral composition according to any one of <1> to <5>, wherein a mass ratio of the component (B) to the component (A), [(B)/(A)], is preferably 1×10⁻⁴ or more and 500×10⁻⁴ or less, more preferably 3×10⁻⁴ or more and 300×10⁻⁴ or less, more preferably 5×10⁻⁴ or more and 100×10⁻⁴ or less.
<8> The oral composition according to any one of <1> to <7>, wherein a content of the component (C) is preferably 0.1 mass ppm or more, more preferably 0.3 mass ppm or more, more preferably 0.7 mass ppm or more, even more preferably 6 mass ppm or less, and is preferably 60 mass ppm or less, more preferably 25 mass ppm or less, more preferably 20 mass ppm or less, even more preferably 15 mass ppm or less.
<9> The oral composition according to any one of <1> to <7>, wherein a content of the component (C) is preferably from 0.1 mass ppm to 60 mass ppm, more preferably from 0.3 mass ppm to 25 mass ppm, more preferably from 0.7 mass ppm to 20 mass ppm, even more preferably from 6 mass ppm to 15 mass ppm.
<10> The oral composition according to any one of <1> to <9>, wherein a mass ratio of the component (C) to the component (B), [(C)/(B)], is preferably 4×10⁻⁴ or more, more preferably 6×10⁻⁴ or more, more preferably 8×10⁻⁴ or more, and is preferably 400×10⁻⁴ or less, more preferably 250×10⁻⁴ or less, more preferably 150×10⁻⁴ or less.
<11> The oral composition according to any one of <1> to <9>, wherein a mass ratio of the component (C) to the component (B), [(C)/(B)], is preferably 4×10⁻⁴ or more and 400×10⁻⁴ or less, more preferably 6×10⁻⁴ or more and 250×10⁻⁴ or less, more preferably 8×10⁻⁴ or more and 150×10⁻⁴ or less.
<12> The oral composition according to any one of <1> to <11>, wherein a mass ratio of the component (C) to the component (A), [(C)/(A)], is preferably 1×10⁻⁶ or more, more preferably 3×10⁻⁶ or more, more preferably 5×10⁻⁶ or more, even more preferably 40×10⁻⁶ or more, and is preferably 300×10⁻⁶ or less, more preferably 180×10⁻⁶ or less, more preferably 120×10⁻⁶ or less, even more preferably 80×10⁻⁶ or less.
<13> The oral composition according to any one of <1> to <11>, wherein a mass ratio of the component (C) to the component (A), [(C)/(A)], is preferably 1×10⁻⁶ or more and 300×10⁻⁶ or less, more preferably 3×10⁻⁶ or more and 180×10⁻⁶ or less, more preferably 5×10⁻⁶ or more and 120×10⁻⁶ or less, even more preferably 40×10⁻⁶ or more and 80×10⁻⁶ or less.
<14> The oral composition according to any one of <1> to <13>, preferably further comprising a lactone as a component (D).
<15> The oral composition according to <14>, wherein the component (D) is preferably one or more selected from the group consisting of γ-butyrolactone, γ-decalactone, and δ-decanolactone, more preferably γ-butyrolactone.
<16> The oral composition according to <14> or <15>, wherein a content of the component (D) is preferably 0.1 mass ppm or more, more preferably 0.5 mass ppm or more, more preferably 1.5 mass ppm or more, and is preferably 90 mass ppm or less, more preferably 60 mass ppm or less, more preferably 40 mass ppm or less.
<17> The oral composition according to <14> or <15>, wherein a content of the component (D) is preferably from 0.1 mass ppm to 90 mass ppm, more preferably from 0.5 mass ppm to 60 mass ppm, more preferably from 1.5 mass ppm to 40 mass ppm.
<18> The oral composition according to any one of <14> to <17>, wherein a mass ratio of the component (D) to the component (B), [(D)/(B)], is preferably 3×10⁻⁴ or more, more preferably 8×10⁻⁴ or more, more preferably 20×10⁻⁴ or more, and is preferably 1 200×10⁻⁴ or less, more preferably 500×10⁻⁴ or less, more preferably 300×10⁻⁴ or less.
<19> The oral composition according to any one of <14> to <17>, wherein a mass ratio of the component (D) to the component (B), [(D)/(B)], is preferably 3×10⁻⁴ or more and 1 200×10⁻⁴ or less, more preferably 8×10⁻⁴ or more and 500×10⁻⁴ or less, more preferably 20×10⁻⁴ or more and 300×10⁻⁴ or less.
<20> The oral composition according to any one of <14> to <19>, wherein a mass ratio of the component (D) to the component (C), [(D)/(C)], is preferably 0.1 or more, more preferably 0.5 or more, more preferably 1.5 or more, and is preferably 50 or less, more preferably 25 or less, more preferably 15 or less.
<21> The oral composition according to any one of <14> to <19>, wherein a mass ratio of the component (D) to the component (C), [(D)/(C)], is preferably from 0.1 to 50, more preferably from 0.5 to 25, more preferably from 1.5 to 15.
<22> The oral composition according to any one of <1> to <21>, wherein the oral composition is preferably an oral composition excluding a coffee beverage.
<23> The oral composition according to any one of <1> to <22>, preferably further comprising furfuryl mercaptan as a component (E), wherein a content of the component (E) is preferably less than 0.00006 mass%, more preferably less than 0.00003 mass%, more preferably less than 0.00001 mass%, and the oral composition is even more preferably substantially free of the component (E).
<24> The oral composition according to any one of <1> to <23>, wherein the component (A) is preferably derived from one or more selected from the group consisting of a lightly roasted coffee bean and a green coffee bean.
<25> The oral composition according to <24>, wherein the lightly roasted coffee bean has an L value of preferably 30 or more, more preferably 32 or more, more preferably 34 or more, more preferably 36 or more, more preferably 38 or more, even more preferably 40 or more, and preferably 60 or less.
<26> The oral composition according to <24>, wherein the lightly roasted coffee bean has an L value of preferably from 30 to 60, more preferably from 32 to 60, more preferably from 34 to 60, more preferably from 36 to 60, more preferably from 38 to 60, even more preferably from 40 to 60.
<27> The oral composition according to any one of <1> to <26>, wherein the oral composition is an oral composition excluding an oral composition using, as a raw material, roasted coffee beans including a roasted coffee bean having an L value of preferably less than 30, more preferably less than 32, more preferably less than 34, more preferably less than 36, more preferably less than 38, even more preferably less than 40.
<28> The oral composition according to any one of <1> to <27>, wherein the oral composition is preferably a solid oral composition.
<29> The oral composition according to <28>, wherein the oral composition is preferably a solid at normal temperature (20°C±15°C).
<30> The oral composition according to <28> or <29>, wherein a form of the oral composition is preferably a powder form, a granule form, a tablet form, a rod form, a plate form, or a block form.
<31> The oral composition according to any one of <28> to <30>, wherein a solid content in the oral composition is preferably 90 mass% or more, more preferably 93 mass% or more, more preferably 95 mass% or more, even more preferably 97 mass% or more.
<32> The oral composition according to any one of <28> to <31>, wherein the oral composition is preferably a food, a pharmaceutical, or a quasi-drug, more preferably a solid food or a powder food.
<33> The oral composition according to any one of <28> to <32>, wherein the oral composition is preferably confectionery, a supplement, a health food, a beauty food, or a dietary supplement, more preferably a supplement.
<34> The oral composition according to any one of <28> to <32>, wherein the oral composition is preferably a granule, a powder, a tablet, a pill, a chewable, or a troche, more preferably a powder, a tablet, or a granule.
<35> The oral composition according to any one of <28> to <34>, preferably further comprising one or more carriers selected from the group consisting of an excipient, a binder, a disintegrant, a lubricant, an extender, a surfactant, a dispersant, a buffer, an antioxidant, a preservative, a quality stabilizer, and a diluent.
<36> The oral composition according to any one of <28> to <35>, wherein the oral composition is preferably an instant beverage composition.

### Examples

### 1. Analysis of Chlorogenic Acid and Caffeine

A HPLC was used as an analyzer. The model numbers of units for forming the apparatus are as described below.
·UV-VIS detector: SPD-20A (manufactured by Shimadzu Corporation)
·Column oven: CTO-20AC (manufactured by Shimadzu Corporation)
·Pump: LC-20AD (manufactured by Shimadzu Corporation)
·Autosampler: SIL-20AC (manufactured by Shimadzu Corporation)
·Column: Cadenza CD-C18, inner diameter of 4.6 mm×length of 150 mm, particle diameter of 3 µm (manufactured by Imtakt Corporation)

Analysis conditions are as described below.
·Sample injection volume: 10 µL
·Flow rate: 1.0 mL/min
·UV-VIS detector set wavelength: 325 nm (chlorogenic acid), 270 nm (caffeine)
·Column oven set temperature: 35°C
·Eluent A: 50 mM acetic acid, 0.1 mM 1-hydroxyethane-1,1-diphosphonic acid, 10 mM sodium acetate, and 5 (v/v)% acetonitrile solution
·Eluent B: acetonitrile

### Concentration Gradient Conditions (vol%)

| Time | Eluent A | Eluent B |
|---|---|---|
| 0.0 min | 100% | 0% |
| 10.0 min | 100% | 0% |
| 15.0 min | 95% | 5% |
| 20.0 min | 95% | 5% |
| 22.0 min | 92% | 8% |
| 50.0 min | 92% | 8% |
| 52.0 min | 10% | 90% |
| 60.0 min | 10% | 90% |
| 60.1 min | 100% | 0% |
| 70.0 min | 100% | 0% |

| | |
|---|---|
| ·3-Caffeoylquinic acid: | 5.3 min |
| ·5-Caffeoylquinic acid: | 8.8 min |
| ·4-Caffeoylquinic acid: | 11.6 min |
| ·3-Feruloylquinic acid: | 13.0 min |
| ·5-Feruloylquinic acid: | 19.9 min |
| ·4-Feruloylquinic acid: | 21.0 min |
| ·3,4-Dicaffeoylquinic acid: | 36.6 min |
| ·3,5-Dicaffeoylquinic acid: | 37.4 min |
| ·4,5-Dicaffeoylquinic acid: | 44.2 min |

The content (mass%) of a chlorogenic acid was determined from an area percentage determined herein by using 5-caffeoylquinic acid (Tokyo Chemical Industry Co., Ltd.) as a standard substance.

| | |
|---|---|
| ·Caffeine: | 19.1 min |

The content (mass%) of caffeine was determined from an area percentage determined herein by using caffeine (FUJIFILM Wako Pure Chemical Corporation) as a standard substance.

### 2. Analysis of 4-Vinylguaiacol and Lactone

A sample was sampled in a vial, and an aroma component in the headspace of the vial was adsorbed to SPME fibers and subjected to GC/MS measurement.

Analysis conditions are as described below.

### HS-GC/MS conditions

·Measuring device: HP 6890 (manufactured by Agilent Technologies, Inc.)
·Column: VF-WAX, inner diameter of 0.25 mm×length of 60 m, film thickness of 0.25 µm (manufactured by GL Sciences Inc.)
·Column temperature: 35°C (4 min)→3°C/min→130°C→5°C/min→240°C (15 min)
·Column pressure: constant flow rate mode (31 kPa)
·Column flow rate: 1.5 mL/min (He)
·Inlet temperature: 240°C
·Injection mode: splitless
·Detector temperature: 200°C
·Carrier gas: helium
·Scan mode: m/z 30 to 500

A commercially available reagent was dissolved in ethanol, and the resultant solution was serially diluted to prepare a standard sample. The standard sample having a predetermined concentration was added to the sample, and the resultant was adsorbed to the SPME fibers in the same manner as with the sample alone, followed by GC/MS measurement. The following m/z peak areas were used in the determination of the amounts of 4-vinylguaiacol, γ-butyrolactone, γ-decalactone, and δ-decanolactone.

·γ-Butyrolactone: 42
·γ-Decalactone: 85
·δ-Decanolactone: 99
·4-Vinylguaiacol: 150

### Production Example 1

### Production of Green Coffee Bean Extract

Green coffee bean extract powder (solid content: 90 g) was dissolved in 360 g of an aqueous solution of ethanol having an ethanol concentration of 60 mass%, and the resultant solution was mixed with 45 g of acid clay (MIZUKA ACE #600, manufactured by Mizusawa Industrial Chemicals, Ltd.), followed by filtration with filter paper precoated with diatomaceous earth. The filtrate was passed through a column filled with 34 mL of coconut shell activated carbon and a column filled with 31 mL of a H-type cation exchange resin to provide a column-treated liquid. The column-treated liquid was concentrated to provide a chlorogenic acid preparation. The content of a chlorogenic acid in the resultant chlorogenic acid preparation was 38 mass%, and the concentration of caffeine therein was 0.0 mass%.

### Examples 1 to 13, Comparative Examples 1 and 2, and Reference Example 1

The respective components shown in Table 1 were uniformly mixed to prepare solid oral compositions, and each of the resultant solid oral compositions was analyzed and subjected to the following sensory evaluation. The results are shown in Table 1.

### Sensory Evaluation

A sensory test was performed after three expert panelists had agreed to use the following evaluation criteria for "astringency" at the time of the ingestion of each of the solid oral compositions obtained in Examples, Comparative Examples, and Reference Example. Then, the final score was determined through discussion on the basis of the scores determined by the respective expert panelists.

### Evaluation Criteria for Astringency

The evaluation was performed in accordance with the following criteria while the score of the astringency of the solid oral composition of Comparative Example 1 was defined as "1" , and the score of the astringency of the solid oral composition of Reference Example 1 was defined as "5".
Score 5: No astringency is sensed.
4: Substantially no astringency is sensed.
3: Astringency is slightly sensed.
2: Astringency is sensed.
1: Astringency is strongly sensed.

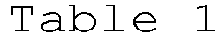

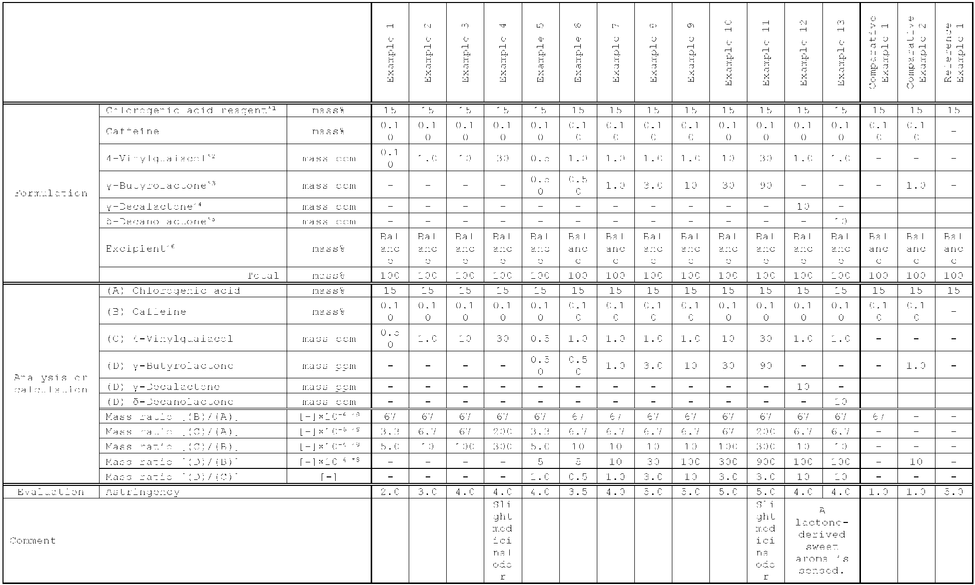

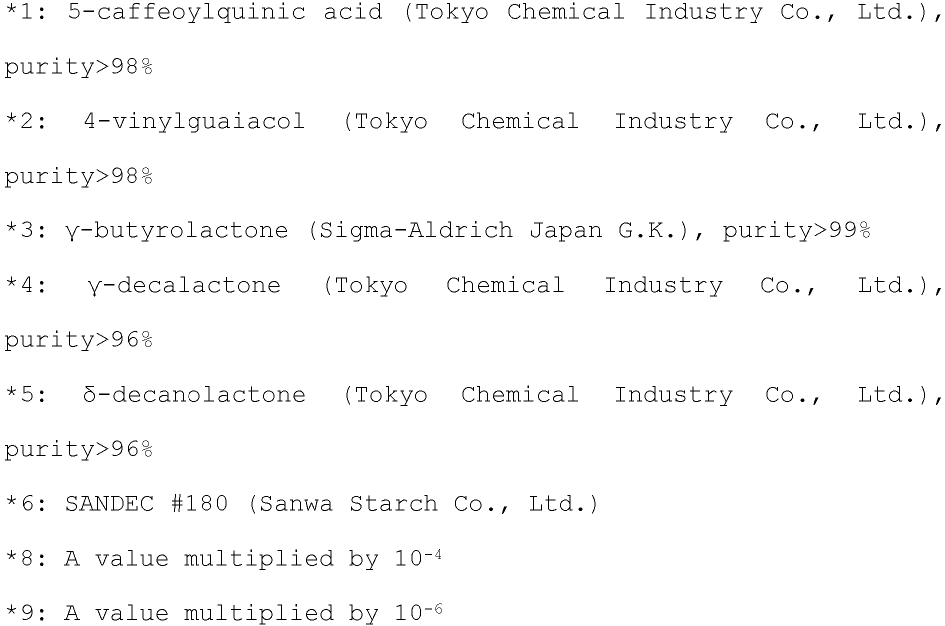

### Examples 14 to 20, Comparative Example 3, and Reference Example 2

The respective components shown in Table 2 were uniformly mixed to prepare solid oral compositions, and each of the resultant solid oral compositions was analyzed and subjected to the following sensory evaluation. The results are shown in Table 2.

### Sensory Evaluation

A sensory test was performed after three expert panelists had agreed to use the following evaluation criteria for "astringency" at the time of the ingestion of each of the solid oral compositions obtained in Examples, Comparative Example, and Reference Example. Then, the final score was determined through discussion on the basis of the scores determined by the respective expert panelists.

### Evaluation Criteria for Astringency

The evaluation was performed in accordance with the following criteria while the score of the astringency of the solid oral composition of Comparative Example 3 was defined as "1", and the score of the astringency of the solid oral composition of Reference Example 2 was defined as "5".
Score 5: No astringency is sensed.
4: Substantially no astringency is sensed.
3: Astringency is slightly sensed.
2: Astringency is sensed.
1: Astringency is strongly sensed.

**Table 2**

| | | | Example 14 | Example 15 | Example 16 | Example 17 | Example 18 | Example 19 | Example 20 | Comparative Example 3 | Reference Example 2 |
|---|---|---|---|---|---|---|---|---|---|---|---|
| Formulation | Green coffee bean extract*⁷ | mass% | 38 | 38 | 38 | 38 | 38 | 38 | 38 | 38 | 38 |
| | Caffeine | mass% | 0.10 | 0.10 | 0.10 | 0.10 | 0.10 | 0.10 | 0.10 | 0.10 | - |
| | 4-Vinylguaiacol*² | mass ppm | 0.50 | 1.0 | 10 | 30 | 1.0 | 1.0 | 1.0 | - | - |
| | γ-Butyrolactone*³ | mass ppm | - | - | - | - | 1.0 | 3.0 | 10 | - | - |
| | Excipient*⁶ | mass% | Balance | Balance | Balance | Balance | Balance | Balance | Balance | Balance | Balance |
| | Total | mass% | 100 | 100 | 100 | 100 | 100 | 100 | 100 | 100 | 100 |
| | (A) Chlorogenic acid | mass% | 14 | 14 | 14 | 14 | 14 | 14 | 14 | 14 | 14 |
| | (B) Caffeine | mass% | 0.10 | 0.10 | 0.10 | 0.10 | 0.10 | 0.10 | 0.10 | 0.10 | - |
| | (C) 4-Vinylguaiacol | mass ppm | 0.7 | 1.2 | 10.2 | 30.2 | 1.2 | 1.2 | 1.2 | 0.2 | 0.2 |
| | Butyrolactone | mass ppm | 0.8 | 0.8 | 0.8 | 0.8 | 1.8 | 3.8 | 10.8 | 0.8 | 0.8 |
| Analysis or calculation | Mass ratio [(B)/(A)] | [-] ×10^{-4 *8} | 71 | 71 | 71 | 71 | 71 | 71 | 71 | 71 | - |
| | Mass ratio [(C)/(A)] | [-] ×10^{-6 *9} | 5.0 | 8.6 | 73 | 216 | 8.6 | 8.6 | 8. 6 | 1.4 | 1.4 |
| | Mass ratio [(C)/(B)] | [-] ×10^{-4 *8} | 7.0 | 12 | 102 | 302 | 12 | 12 | 12 | 2 | - |
| | Mass ratio [(D)/(B)] | [-] ×10^{-4 *8} | 8.0 | 8.0 | 8.0 | 8.0 | 18 | 38 | 108 | 8.0 | - |
| | Mass ratio [(D)/(C)] | [-] | 1.1 | 0.67 | 0.078 | 0.027 | 1.5 | 3.2 | 9.0 | 4.0 | 4.0 |
| Evaluation | Astringency | | 2.0 | 3.0 | 4.0 | 4.0 | 4.0 | 5.0 | 5.0 | 1.0 | 5.0 |
| Comment | | | | | | Slight medicinal odor | | | | | |

| | | | | | | | | | | | |
|---|---|---|---|---|---|---|---|---|---|---|---|
| *2: 4-vinylguaiacol (Tokyo Chemical Industry Co., Ltd.), purity>98% *3: γ-butyrolactone (Sigma-Aldrich Japan G.K.) *6: SANDEC #180 (Sanwa Starch Co., Ltd.) *7: The green coffee bean extract obtained in Production Example 1: chlorogenic acid content: 38 mass%, 4-vinylguaiacol: 0.5 mass ppm, γ-butyrolactone: 2 mass ppm *8: A value multiplied by 10⁻⁴ *9: A value multiplied by 10⁻⁶ | | | | | | | | | | | |

### Examples 21 to 23, Comparative Example 4, and Reference Example 3

The respective components shown in Table 3 were uniformly mixed to prepare solid oral compositions, and each of the resultant solid oral compositions was analyzed and subjected to the following sensory evaluation. The results are shown in Table 3.

### Sensory Evaluation

A sensory test was performed after three expert panelists had agreed to use the following evaluation criteria for "astringency" at the time of the ingestion of each of the solid oral compositions obtained in Examples, Comparative Example, and Reference Example. Then, the final score was determined through discussion on the basis of the scores determined by the respective expert panelists.

### Evaluation Criteria for Astringency

The evaluation was performed in accordance with the following criteria while the score of the astringency of the solid oral composition of Comparative Example 4 was defined as "1", and the score of the astringency of the solid oral composition of Reference Example 3 was defined as "5".
Score 5: No astringency is sensed.
4: Substantially no astringency is sensed.
3: Astringency is slightly sensed.
2: Astringency is sensed.
1: Astringency is strongly sensed.

**Table 3**

| | | | Example 21 | Example 22 | Example 23 | Comparative Example 4 | Reference Example 3 |
|---|---|---|---|---|---|---|---|
| Formulation | Green coffee bean extract*⁷ | mass% | 38 | 38 | 38 | 38 | 38 |
| | Caffeine | mass% | 0.010 | 0.010 | 0.010 | 0.010 | - |
| | 4-Vinylguaiacol^{*2} | mass ppm | 1.0 | 10 | 1.0 | - | - |
| | γ-Butyrolactone^{*3} | mass ppm | - | - | 10 | - | - |
| | Excipient^{*6} | mass% | Balance | Balance | Balance | Balance | Balance |
| | Total | mass% | 100 | 100 | 100 | 100 | 100 |
| | (A) Chlorogenic acid | mass% | 14 | 14 | 14 | 14 | 14 |
| | (B) Caffeine | mass% | 0.010 | 0.010 | 0.010 | 0.010 | - |
| | (C) 4-Vinylguaiacol | mass ppm | 1.2 | 10.2 | 1.2 | 0.2 | 0.2 |
| | (D) γ-Butyrolactone | mass ppm | 0.8 | 0.8 | 10.8 | 0.8 | 0.8 |
| Analysis or calculation | Mass ratio [(B)/(A)] | [-] ×10^{-4 *8} | 7.1 | 7.1 | 7.1 | 7.1 | - |
| | Mass ratio [(C)/(A)] | [-] ×10^{-6 *9} | 8.6 | 73 | 8. 6 | 1.4 | 1.4 |
| | Mass ratio [(C)/(B)] | [-] ×10^{-4 *8} | 120 | 1 020 | 120 | 20 | - |
| | Mass ratio [(D)/(B)] | [-] ×10^{-4 *8} | 80 | 80 | 1 080 | 80 | - |
| | Mass ratio [(D)/(C)] | [-] | 0.66 | 0.078 | 9.0 | 4.0 | 4.0 |
| Evaluation | Astringency | | 3.0 | 4.0 | 5.0 | 1.0 | 5.0 |

| | | | | | | | |
|---|---|---|---|---|---|---|---|
| *2: 4-vinylguaiacol (Tokyo Chemical Industry Co., Ltd.), purity>98% *3: γ-butyrolactone (Sigma-Aldrich Japan G.K.) *6: SANDEC #180 (Sanwa Starch Co., Ltd.) *7: The green coffee bean extract obtained in Production Example 1: chlorogenic acid content: 38 mass%, 4-vinylguaiacol: 0.5 mass ppm, γ-butyrolactone: 2 mass ppm *8: A value multiplied by 10⁻⁴ *9: A value multiplied by 10⁻⁶ | | | | | | | |

### Examples 24 to 26, Comparative Example 5, and Reference Example 4

The respective components shown in Table 4 were uniformly mixed to prepare solid oral compositions, and each of the resultant solid oral compositions was analyzed and subjected to the following sensory evaluation. The results are shown in Table 4.

### Sensory Evaluation

A sensory test was performed after three expert panelists had agreed to use the following evaluation criteria for "astringency" at the time of the ingestion of each of the solid oral compositions obtained in Examples, Comparative Example, and Reference Example. Then, the final score was determined through discussion on the basis of the scores determined by the respective expert panelists.

### Evaluation Criteria for Astringency

The evaluation was performed in accordance with the following criteria while the score of the astringency of the solid oral composition of Comparative Example 5 was defined as "1", and the score of the astringency of the solid oral composition of Reference Example 4 was defined as "5".
Score 5: No astringency is sensed.
4: Substantially no astringency is sensed.
3: Astringency is slightly sensed.
2: Astringency is sensed.
1: Astringency is strongly sensed.

**Table 4**

| | | | Example 24 | Example 25 | Example 26 | Comparative Example 5 | Reference Example 4 |
|---|---|---|---|---|---|---|---|
| Formulation | Green coffee bean extract*⁷ | mass% | 20 | 20 | 20 | 20 | 20 |
| | Caffeine | mass% | 0.10 | 0.10 | 0.10 | 0.10 | - |
| | 4-Vinylguaiacol^{*2} | mass ppm | 1.0 | 10 | 1.0 | - | - |
| | γ-Butyrolactone^{*3} | mass ppm | - | - | 10 | - | - |
| | Excipient^{*6} | mass% | Balance | Balance | Balance | Balance | Balance |
| | Total | mass% | 100 | 100 | 100 | 100 | 100 |
| | (A) Chlorogenic acid | mass% | 7. 6 | 7. 6 | 7. 6 | 7. 6 | 7.6 |
| | (B) Caffeine | mass% | 0.10 | 0.10 | 0.10 | 0.10 | - |
| | (C) 4-Vinylguaiacol | mass ppm | 1.1 | 10.1 | 1.1 | 0.1 | 0.1 |
| | (D) γ-Butyrolactone | mass ppm | 0.4 | 0. 4 | 10.4 | 0. 4 | 0.4 |
| Analysis or calculation | Mass ratio [(B)/(A)] | [-] ×10^{-4 *8} | 132 | 132 | 132 | 132 | - |
| | Mass ratio [(C)/(A)] | [-] ×10^{-6 *9} | 14 | 133 | 14 | 1.3 | 1.3 |
| | Mass ratio [(C)/(B)] | [-]×10^{-4 *8} | 11 | 101 | 11 | 1.0 | - |
| | Mass ratio [(D)/(B)] | [-] ×10^{-4 *8} | 4.0 | 4.0 | 104 | 4.0 | - |
| | Mass ratio [(D)/(C)] | [-] | 0.36 | 0.040 | 9.5 | 4.0 | 4.0 |
| Evaluation | Astringency | | 3.0 | 4.0 | 5.0 | 1.0 | 5.0 |

| | | | | | | | |
|---|---|---|---|---|---|---|---|
| *2: 4-vinylguaiacol (Tokyo Chemical Industry Co., Ltd.), purity>98% *3: γ-butyrolactone (Sigma-Aldrich Japan G.K.) *6: SANDEC #180 (Sanwa Starch Co., Ltd.) *7: The green coffee bean extract obtained in Production Example 1: chlorogenic acid content: 38 mass%, 4-vinylguaiacol: 0.5 mass ppm, γ-butyrolactone: 2 mass ppm *8: A value multiplied by 10⁻⁴ *9: A value multiplied by 10⁻⁶ | | | | | | | |

It found from Tables 1 to 4 that the astringency occurring at the time of the incorporation of a trace amount of caffeine into a high concentration of the chlorogenic acid can be suppressed by incorporating 4-vinylguaiacol at a predetermined quantitative ratio with respect to caffeine. Further, it is found that the astringency-suppressing effect is not exhibited by the lactone alone, but is enhanced by incorporating the lactone together with 4-vinylguaiacol.

## Claims

1. An oral composition, comprising the following components (A), (B), and (C):
(A) 5 mass% to 20 mass% of a chlorogenic acid;
(B) 0.001 mass% or more and less than 0.4 mass% of caffeine; and
(C) 4-vinylguaiacol,
wherein a mass ratio of the component (C) to the component (B), [(C)/(B)], is 3×10⁻⁴ or more and 1100×10⁻⁴ or less.

2. The oral composition according to claim 1, wherein a mass ratio of the component (C) to the component (A), [(C)/(A)], is 1×10⁻⁶ or more and 300×10⁻⁶ or less.

3. The oral composition according to claim 1 or 2, wherein a mass ratio of the component (B) to the component (A), [(B)/(A)], is 1×10⁻⁴ or more and 500×10⁻⁴ or less.

4. The oral composition according to any one of claims 1 to 3, wherein a content of the component (C) is from 0.1 mass ppm to 60 mass ppm.

5. The oral composition according to any one of claims 1 to 4, further comprising a lactone as a component (D).

6. The oral composition according to claim 5, wherein the component (D) comprises γ-butyrolactone.

7. The oral composition according to claim 5 or 6, wherein a mass ratio of the component (D) to the component (B), [(D)/(B)], is 3x10⁻⁴ or more and 1200×10⁻⁴ or less.

8. The oral composition according to any one of claims 5 to 7, wherein a mass ratio of the component (D) to the component (C), [(D)/(C)], is from 0.1 to 50.

9. The oral composition according to any one of claims 1 to 8, wherein the oral composition is a solid oral composition.

10. The oral composition according to any one of claims 1 to 8, wherein the oral composition is a liquid oral composition.
